# EUROPEAN PATENT APPLICATION

(11) **EP 2 565 278 A1**
(43) Date of publication of application: **06.03.2013**
(21) Application number: 11774452.4
(22) Date of filing: 26.04.2011
(51) Int. Cl.: C12Q 1/68, C12N 15/113, B01J 19/10, A61K 31/713, A61K 48/00, A61P 35/00

(54) **METHOD FOR THE DELIVERY OF OLIGONUCLEOTIDES**

(30) Priority: 28.04.2010 ES 201030629
(71) Applicant: Consejo Superior De Investigaciones Científicas (CSIC), 28006 Madrid (ES); Universidad De Barcelona, 08028 Barcelona (ES)
(72) Inventor: ERITJA CASADELLÁ, Ramón, E-08034 Barcelona (ES); OCAMPO, Sandra Milena, E-08034 Barcelona (ES); BLASCO SOLE, Francesc Xavier, E-08034 Barcelona (ES); PERALES LOSA, José Carlos, E-08028 Barcelona (ES)
(74) Representative: Ungria López, Javier
(86) International application number: PCT/ES2011/070295
(87) International publication number: WO 2011/135140

(57) **Abstract**

The invention relates to a method for obtaining formulations which improve the binding capacity of siRNAs in relation to plasma components, which promote the transport of siRNA in the blood, which increase the passage of siRNA through the cell membrane and, consequently, which increase the inhibitory activity of siRNA. The invention also relates to a method for obtaining a formulation comprising siRNA associated with plasma components, characterised in that the method includes a step in which the plasma components are dispersed in an aqueous medium.

SEQUENCE LISTING

<110> Superior Council of Scientific Research, CSIC,
and University of Barcelona

<120> Method for the delivery of oligonucleotides

<130> ES1641.514

<160> 7
<170> PatentIn version 3.5

<210> 1
<211> 21
<212> DNA
<213> Artificial sequence

<220>
<223> Fragment of the guide or antisense sequence of the Mus
musculus TNF alpha RNA to which two thymines have been added
in the 3' end.

<400> 1
gaggcugaga cauaggcact t                                                21

<210> 2
<211> 21
<212> DNA
<213> Artificial sequence

<220>
<223> Fragment of the complementary or sense sequence of the
Mus musculus TNF alpha RNA to which two thymines have been
added in the 3' end.

<400> 2
gugccuaugu cucagccuct t                                                21

<210> 3
<211> 21
<212> DNA
<213> Artificial sequence

<220>
<223> Fragment of a guide or antisense sequence of Euglena
gracilis RNA to which two thymines have been added in the 3'
 end.

<400> 3
cagucgcguu ugcgacuggt t                                                21

<210> 4
<211> 21
<212> DNA
<213> Artificial sequence

<220>
<223> Fragment of a complementary or sense sequence of
Euglena gracilis RNA to which two thymines have been added
in the 3' end.

<400> 4
ccagucgcaa acgcgacugt t                                                21

<210> 5
<211> 21
<212> DNA
<213> Artificial sequence

<220>

<223> Fragment of the guide or antisense sequence of the
Mus musculus PEPCK-L RNA to which two thymines have been
added in the 3' end.

<400> 5
uuacaucugg cugauucuct t                                                21

<210> 6
<211> 21
<212> DNA
<213> Artificial sequence

<220>
<223> Fragment of the complementary or sense sequence of
the Mus musculus PEPCK-L RNA to which two thymines have been
added in the 3' end.

<400> 6
gagaaucagc cagauguaat t                                                21

<210> 7
<211> 21
<212> DNA
<213> Artificial sequence

<220>
<223> Fragment of the complementary or sense sequence of
the Mus musculus TNF alpha RNA to which two thymines have
 been added in the 3' end and some nucleotides have been
methylated.

<220>
<221> misc_RNA
<222> 5..5
<223> The nucleotide defined as "n" corresponds to a "cm"
nucleotide in accordance with the list of modified
nucleotides of table 2 of appendix 2 of the Standard ST.25

<220>
<221> misc_feature
<222> 5..5
<223> n is a, c, g, t or u

<220>
<221> misc_RNA
<222> 10..10
<223> The nucleotide defined as "n" corresponds to a
 "um" nucleotide in accordance with the list of modified
 nucleotides of table 2 of appendix 2 of the Standard ST.25

<220>
<221> misc_feature
<222> 10..11
<223> n is a, c, g, t or u

<220>
<221> misc_RNA
<222> 11..11
<223> The nucleotide defined as "n" corresponds to a
 "cm" nucleotide in accordance with the list of modified
nucleotides of table 2 of appendix 2 of the Standard ST.25

<220>
<221> misc_RNA
<222> 13..13
<223> The nucleotide defined as "n" corresponds to a "cm"
nucleotide in accordance with the list of modified nucleotides
 of table 2 of appendix 2 of the Standard ST.25

<220>

<221> misc_feature
<222> 13..13
<223> n is a, c, g, t or u

<220>
<221> misc_RNA
<222> 16..16
<223> The nucleotide defined as "n" corresponds to a "cm"
 nucleotide in accordance with the list of modified nucleotides
of table 2 of appendix 2 of the Standard ST.25

<220>
<221> misc_feature
<222> 16..16
<223> n is a, c, g, t or u

<220>
<221> misc_RNA
<222> 18..18
<223> The nucleotide defined as "n" corresponds to a "um"
nucleotide in accordance with the list of modified nucleotides
 of table 2 of appendix 2 of the Standard ST.25

<220>
<221> misc_feature
<222> 18..18
<223> n is a, c, g, t or u

<400> 7
gugcnuaugn nunagncnct t                                                21

## Description

The present invention relates to a method for the manufacture of compositions comprising siRNA associated with plasma components. It therefore belongs to the field of biotechnology.

### STATE OF THE PRIOR ART

RNA interference (RNAi) is an important mechanism for gene regulation that can be used for the specific silencing of genes. The process is started by double-stranded RNAs called siRNAs (small interfering RNAs). The siRNAs, complementary to a certain messenger RNA, bind to a protein complex known as RISC. The complex formed by the binding of the antisense or guide strand with RISC catalyzes the efficient degradation of a specific messenger RNA, causing a decline in the target protein. During the past years, numerous studies have been carried out with the purpose of using this phenomenon of natural gene regulation as the basis for a new therapy aimed at the specific reduction of a previously determined protein. Thus, said therapy could be used for the treatment of diseases which are known to be caused by the over expression of genes such as cancer or inflammatory diseases [D. Brumcot et al. RNAi therapeutics: a potential new class of pharmaceutical drugs. Nature Chemical Biology 2 (2006), pages 711-719; A. de Fougerolles et al. RNA interference in vivo: toward synthetic small inhibitory RNA-based therapeutics Methods in Enzymology 392 (2005), pages 278-296; C. Chakraborty Potentiality of small RNA interference (siRNA) as recent therapeutic targets for gene-silencing. Current Drug Targets 8 (2007) pages. 469-482].

The *in vivo* cell delivery of siRNAs is perhaps one of the most important problems for the development of an efficient therapeutic drug based on RNA interference. [D. Brumcot et al. RNAi therapeutics: a potential new class of pharmaceutical drugs. Nature Chemical Biology 2 (2006), pages 711-719; A. de Fougerolles et al. RNA interference in vivo: toward synthetic small inhibitory RNA-based therapeutics Methods in Enzymology 392 (2005), pages 278-296; C. Chakraborty Potentiality of small RNA interference (siRNA) as recent therapeutic targets for gene-silencing. Current Drug Targets 8 (2007) pages. 469-482]. siRNAs, which are negatively charged hydrophilic molecules do not have the ability, by themselves, to circulate in the blood and being internalized, or to penetrate into the cell through the cell membrane which is hydrophobic. To solve this problem several strategies such as the use of nanoparticles or the use of liposomes have been described [J. Soutschek et al. Therapeutic silencing of an endogenous gene by systemic delivery of modified siRNAs. Nature 432, (2004) pages 173-178; D.H. Kim et al. Strategies for silencing human disease using RNA interference. Nature Reviews 8 (2007), pages 173-184]. These vehicles may have a dual function: to be cell permeating agents and, in turn, to be siRNAs protective agents, since the naked siRNAs are rapidly degraded by the RNAses in serum. For this reason, a large number of modified siRNAs have been designed and synthesized to make the most of the advantage of the binding of the modified siRNAs to specific components of the serum (such as albumin, HDL and LDL) and, in this way, help the distribution of the siRNAs molecules in the body.

### DESCRIPTION OF THE INVENTION

The present invention is related to a method for obtaining formulations that improve the binding capacity of the siRNAs to the plasma components, favouring the transport of the siRNAs in the blood, which increase the passage of the siRNAs through the cell membrane, and thus increase the inhibitory activity of the siRNAs. The formulations obtained by the process of the invention clearly improve the entry of the siRNAs in cell cultures and the biodistribution of the siRNA in the tissues. This action is produced by the binding of the siRNAs to the plasma components, such as the saturable sites of plasma proteins such as albumin, or glycerides such as HDL or LDL, that allow the siRNA to reach the different tissues, to remain and be detected even after 4 hours of the intravenous administration of the siRNA in mice.

Therefore a first aspect of the invention is a process for obtaining a formulation comprising siRNA associated with plasma components, characterized in that the process comprises one step in which the plasma components are dispersed in aqueous medium.

A second aspect of the present invention are the pharmaceutical compositions of siRNA associated with plasma components obtainable by any of the processes defined in the first aspect or in any of its particular embodiments.
A final aspect of the present invention is the use of the pharmaceutical compositions according to any of the compositions of the second aspect for the preparation of a medicament.

### DETAILED DESCRIPTION OF THE INVENTION

The present inventors have discovered that, surprisingly, the plasma components, in particular plasma proteins and plasma lipids are capable of associating with siRNA, forming compounds, or complexes that protect the siRNA from the degradation by the RNAses. For this it is vital that these plasma components are dispersed, or disgregated. There are different techniques known in the art to carry out this dissociation, but one of the used ones, due to its versatility, economy and ease is through the use of ultrasound, whether by applying the ultrasound source directly on the solution or by using a bath equipped with an ultrasound source. [Japanese Journal of Applied Physics, 47, 2008 pages 2000-2004].

The ratio between siRNA and the plasma components may vary between 1:100 and 1:50000 p/p, but preferably it varies between 1:500 and 1:10000. The ratios that vary between 1:3000 and 1:5000 are preferred, because high concentrations of siRNA are obtained and it shows good stability.

Each of the strands of the siRNA duplex preferably comprises between 15 and 40 nucleotides, related to the pairs of nucleotides since it is a double-stranded RNA to achieve entering into the cell and most preferably between 19 and 25 nucleotides.

The dispersion can be carried out at different temperature ranges, but the optimal temperatures would be between 0 and 85 °C, more preferably between 5 and 50 °C, and even more preferably between 10 and 35 °C. With respect to the duration of the dispersion step it depends on the effectiveness of the method used, but usually, and especially when it is dispersed through ultrasound, it lasts at least 1 second, more preferably at least 15 seconds, even more preferably between 30 seconds and 2 hours, and still more preferably between 60 seconds and 30 min.

The dispersion, or disintegration, can be carried out prior to the mixing of the plasma components with siRNA. Another alternative which is in turn preferred, is to carry out the dispersion of the plasma components in the presence of siRNA, whereby the association is carried out just after they have been dispersed, so that it prevents or/and reduces that the plasma components are aggregated again.

As already mentioned the association between the dispersed plasma components and the siRNA improve the stability of the product. These plasma components can be obtained from different methods known in the art, as for example lyophilisation of blood plasma or by ultrafiltration thereof, although plasma and/or serum can be used as source of the plasma components, directly without any previous treatment. This is surprising because the siRNA is unstable in the plasma *per se,* but this siRNA shows a totally different behaviour if the plasma has been dispersed. This is useful because it facilitates the manufacturing process, since the isolation of the plasma components is not necessary.

The process of the invention can be used for manufacturing various types of formulations, but they have shown good absorption and biodistribution results when these are injectable.

Another advantage of this process is that the improvement in the absorption and biodistribution is not limited to a type of siRNA in particular. For example, forming associations with siRNA without the need to be derivatized in some of its 3' or 5'-terminal positions has been achieved, such as for example in the unmodified siRNA synthesized by chemical methods or synthesized from a template DNA through the use of RNA polymerase. Although it has been observed that the stability of siRNA can also be improved when it is derivatized at its 3' or 5' position with lipids, peptides or carbohydrates, or another organic molecule that delays the degradation of the siRNA by nucleases, in any of its strands, the guide or the complementary strand. Additionally the sense or antisense strand can incorporate certain compounds for improving the entry of the molecule in the cell, such as the introduction of lipophilic compounds such as cholesterol, o fatty acids, etc.

The siRNA duplex may comprise derivatization such as e.g. substitutions selected from 2'-O-methyl-ribonucleotide or 2'-deoxyribonucleotide or 2'-methoxyethyl-ribonucleotide or 2'-fluoro-deoxyribonucleotide, or 2'-fluoro-arabinonucleotide, conformationally restricted nucleosides (as those known with the acronyms LNA or HNA), RNA with phosphorothioate bonds, or abasic moieties or ribitol or nucleosides containing modified bases such as 5-methylcytosine, 5-methyluracil, 4-alkynylcytosine, 5-alkynyluracil, 5-halogencytosine, 5-halogenuracil, or other modified pyrimidines, 7-deazadenine, 7-deazaguanine, hypoxanthine or other modified purines. The derivatization may incorporate any of the two strands, although the complementary strand since is more permissive to changes.

The siRNA duplex (Y) can inhibit and/or silence, this inhibition/silencing being total or partial with respect to a control, various genes of pharmacological interest such as the tumor necrosis factor alpha (TNFα), vascular endothelial growth factor (VEGF), VEGF receptor (VEGF R1/2), granulocyte (GM-CSF-1) and macrophages (M-CSF-1) colony suppressor factor, angiopoietin (ANGPT), apolipoprotein (ApoB), choroidal neovascularization (CNV), phosphoenolpyruvate carboxykinase (PEPCK), human epidermal growth factor receptor (HER2), macrophage inflammatory protein (MIP2), N-methyl-D aspartate receptor (NMDA), keratinocyte-derived cytokines (KC), delta opioid receptor (DOR), Discoidin domain receptor (DDR1), PIV phosphoprotein gene (PIV-P), heme oxygenase (HMOX1), caveloin, dopamine transporter, Green fluorescent protein and/or Swing sarcoma protein (EWS-FL/1). The preferred genes for silencing/inhibiting are the tumor necrosis factor alpha (TNFα), vascular endothelial growth factor (VEGF), VEGF receptor (VEGF R1/2), granulocyte (GM-CSF-1) and macrophages (M-CSF-1) colony suppressor factor. TNF-α is an important mediator of the apoptosis both in inflammation and immune response. In addition, the over expression of TNF-α is confirmed in the triggering of the pathogenesis of many human diseases. Therefore the inhibition of this cytokine is relevant from the biomedical point of view. PEPCK is an important protein in the process of gluconeogenesis and is over expressed in diabetes. It is responsible for an increase in the production of hepatic glucose in diabetic patients and in animal models.

As examples of the present invention the siRNA sequence is: 1) antisense or guide anti- TNFα 5'-GAG GCU GAG ACA UAG GCA C-dT-dT-3' (SEQ ID NO: 1) and 2) sense or complementary anti- TNFα: 5'-GUG CCU AUG UCU CAG CCU C-dT-dT-3' (SEQ ID NO: 2). The RNA monomers are indicated in capital letters, dT represents thymidine residues. This siRNA duplex (anti TNF-α) had already been described for efficiently regulating the mouse TNF-α mRNA (D.R. Sorensen et al. Gene silencing by systemic delivery of synthetic siRNA in adult mice. Journal of Molecular Biology 327 (2003) pages 761-766.). 12 duplex have been prepared with various hydrophobic compounds at the ends either in the guide strand or in the complementary strand (Table 1). Also 3) anti-PEPCK-C antisense or guide strand: 5'-UUACAUCUGGCUGAUUCUC-dT-dT-3' (SEQ ID NO: 5) and 4) anti-PEPCK-C sense or complementary strand: 5'-GAGAAUCAGCCAGAUGUAA-dT-dT-3' (SEQ ID NO: 6). The RNA monomers are indicated in capital letters, dT represents thymidine residues.

The pharmaceutical compositions of the second aspect of the invention comprise a preferred embodiment of at least one pharmaceutically acceptable excipient or vehicle. The excipients include any inert or non-active material or used in the preparation of a pharmaceutical dosage form. For example, the tablet excipients include, but are not limited to: calcium phosphate, cellulose, starch or lactose. The liquid dosage forms also include oral liquids for example in the form of spirits or suspensions, as well as injectable solutions. The pharmaceutical composition may be formulated for transdermal administration in patch form. All the compositions described above may contain optionally one or more of each of the following excipients: vehicles, diluents, colourings, flavouring agents, lubricants, solubilising agents, disintegrating agents, binders and preservatives.

Although one of the advantages of the present invention is that siRNA can be formulated without the need of a transfection agent, this can be added to the pharmaceutical composition for improving its properties, or for vectorizing it. The preferred transfection agents are selected from one or mixtures of the following compounds: lipofectin, lipofectamine, oligofectamine, effectene, cellfectin, DOTAP, DOPE, fugene, polyethylene glycol, cholesterol, polyethylenimide (PEI), Jet-polyethylenimide, cell penetrating peptides, trojan peptides, TAT peptide, penetratin, oligoarginine, poly-lysine, rabies virus glycoprotein, gold nanoparticles, dendrimers, carbon nanotubes, cationic lipids and liposomes.

The pharmaceutical compositions described in the present specification may have several uses, but are preferred for the treatment of cancer, inflammation, colitis, ulcerative colitis, Crohn's disease and rheumatoid arthritis. In general, the compositions of the present invention are useful for the preparation of medicaments for gene silencing.

The preferred pharmaceutical composition for the present invention is presented in a form adapted for oral or parenteral administration, preferably parenteral.

The administration of the compounds of this invention can be via any procedure that releases the compound, preferably to the desired tissue. These procedures include the oral, intravenous, intramuscular, subcutaneous or intramedullary, intraduodenal routes, etc. Preferably the pharmaceutical composition of the present invention can be administered locally by injection in an area close to the region of interest (intramuscularly, subcutaneously, intradermally), injection in an area close to the region of interest or intravenous injection.

With the purpose of the parenteral administration, they can be used as well as sterile aqueous solutions. If necessary, such aqueous solutions can be buffered adequately, and the liquid extender was first converted into isotonic with the sufficient saline or glucose. These aqueous solutions are especially suitable for the purposes of intravenous, intramuscular, subcutaneous and intraperitoneal injection. In this regard, all sterile aqueous means used can be obtained easily by standard techniques well known by those persons skilled in the art.

### Definitions

The blood plasma is the yellowish liquid component of the blood, in which the blood cells should be normally suspended. It represents 55% of the total blood volume. The greater proportion is water (90% of the volume) and contains dissolved proteins, glucose, clotting factors, mineral ions, hormones and carbon dioxide (the principal medium for the transport of excreted products being the plasma) and other components.

The blood plasma is prepared by centrifugation of a tube of fresh blood, until the cells precipitate at the bottom of the tube. The blood plasma has an approximate density of 1025 kg/m³ or 1.025 kg/L. The blood serum is blood plasma without Fibrinogen or other clotting factors.

The plasma contains a wide variety of proteins including albumin, immunoglobulins and clotting proteins such as Fibrinogen. Albumin constitutes about 60% of the total of the plasma proteins and it is present in concentrations between 35 and 55 mg/ml. The plasma is the main contributor of the osmotic pressure of the blood and works as carrier molecule for other molecules with low aqueous solubility such as hormones soluble in lipids, enzymes, fatty acids, metal ions, pharmaceutical compounds. Albumin is structurally stable due to its 17 disulfide bridges and is the only one that has high solubility in water and has the lowest isoelectric potential (ip) of plasma proteins. Due to its structural integrity it remains stable under conditions in which other proteins would be denatured.

The plasma is formed by 82.5% water, in addition to many inorganic and organic substances (plasma solutes), distributed in the following way: Plasma proteins (70%): Fibrinogen (7%), immunoglobulins (38%), albumin (54%), other proteins (1%): VLDL, LDL, HDL, Prothrombin, transferrin. Organic metabolites (non electrolytic) and waste compounds (20%), fosfolipids (280 mg/dL), cholesterol (150 mg/dL), triacylglycerols (125 mg/dL), glucose (100 mg/dL), urea (15 mg/dL), lactic acid (10 mg/dL), uric acid (3 mg/dL), creatinine (1.5 mg/dL), bilirubin (0.5 mg/dL) and bile salts (traces).

siRNAs means in the context of the present invention small fragments of double-stranded RNA interference of synthetic or natural origin complementary to the sequence of the genes that are used for the specific inhibition of the genes to which they are complementary. The siRNAs are formed by two strands of RNA called guide strand (or antisense strand) and complementary strand (or sense strand). The guide strand of the siRNA binds to a protein complex called RNA interference silencing complex (abbreviated as "RISC") and the resulting complex is responsible for the cellular degradation of messenger RNA complementary to the guide strand. For the binding of the guide strand to the RISC complex it is necessary to administer the double-stranded RNA or siRNA since the guide single strand by itself cannot bind the RISC complex.

siRNAs associated with plasma components means in the context of the present invention the different complexes formed between the siRNAs and the molecules naturally present in the plasma formed after a process of disintegration or dispersion of the plasma components. In the process of association are mainly involved electrostatic interactions and hydrophobic interactions with the plasma components that are mostly blood proteins (albumin, Fibrinogen, immunoglobulins, HDL, VLDL, LDL, Prothrombin, transferrin etc.) and secondly lipids naturally occurring in the plasma (triglycerides, cholesterol, fosfolipids, bile acids, etc.).

Plasma components means the molecules that are naturally dissolved in the plasma, and in particular the mix of proteins and lipids present in the plasma. Due to the composition of the plasma this mixture may contain Fibrinogen, immunoglobulins, albumin and other proteins such as VLDL, LDL, HDL, Prothrombin, transferrin, etc., along with triglycerides, cholesterol, fosfolipids, bile acids and etc. The ratios between the plasma components for use in the present invention, and in particular proteins and lipids may vary and can be adjusted according to need. These components may consist basically of: plasma proteins; or plasma lipids; although they can also be found in the form of a mixture of protein vs. lipids ratios of between 1:1000 and 1000:1. In a preferred embodiment, the ratio of proteins to plasma lipids is around the relationship in which it is found in the plasma.

Dispersing the plasma components in aqueous medium in the context of the present invention means the application of physical force such as the one produced by the action of ultrasound, to the solution comprising the plasma components, such as e.g. the own plasma, which results in the separation of the molecular aggregates to give more or less isolated molecules exposing the hydrophobic pockets and the charged parts that were occluded by the process of aggregation to the components of the aqueous solution, which results in an increase of the forming capacity of electrostatic interactions and hydrophobic interactions with the siRNAs. The degree of dispersion of the plasma components could be estimated by various techniques that are based on the phenomenon of light scattering by the biomolecules and that allow to estimate the hydrodynamic size of the biomolecules.

The term "gene expression" means the cellular production of a particular protein encoded by the corresponding gene. Generally, the gene that is located in the chromosomes present in the nucleus of the cells is transcribed generating a molecule of messenger RNA that is released into the cytoplasm. There, the sequence of the messenger RNA directs the synthesis of the protein. The result of this process, which is known as gene expression, is the synthesis of a protein the sequence of which is encoded by the corresponding gene.

### DESCRIPTION OF THE FIGURES

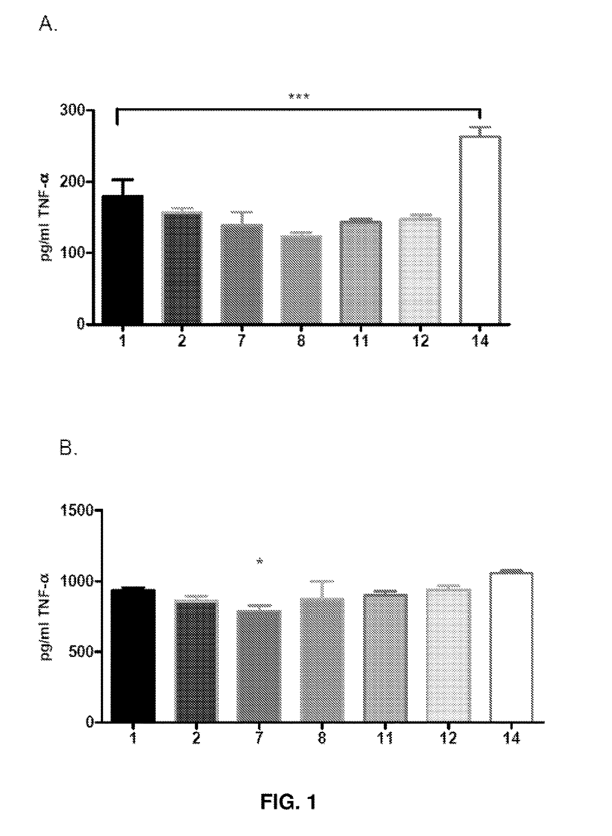

FIGURE 1 shows the *in vivo* inhibitory activity of several siRNAs conjugated with C₁₄ and C₁₈ lipids against mouse TNF-α, using protocols A and B in HeLa cells. 1. Unmodified siRNA, 2. siRNA conjugated with cholesterol in the 3' end of the complementary strand, 7. siRNA conjugated with a C₁₄ lipid in the 3' end of the complementary strand, 8. siRNA conjugated with a C₁₈ lipid the 3' end of the complementary strand, 11. siRNA conjugated with a C₁₄ lipid in the 3' end of the complementary strand and 2'OMe units, 12. siRNA conjugated with C₁₄N in the 5' end of the complementary strand, 14. siRNA with random sequence used as negative control sequence. The description of the sequences 1, 2, 7, 8, 11, 12 and 14 of the siRNAs is detailed in Table 1 and the chemical structure of the changes is shown in schemes 1 and 2. The data represent the means ± SE, n=3 and are compared with the random sequence. ***p<0.001, *p<0.05. The statistical analysis was carried out using the ANOVA method with the Bonferroni treatment.

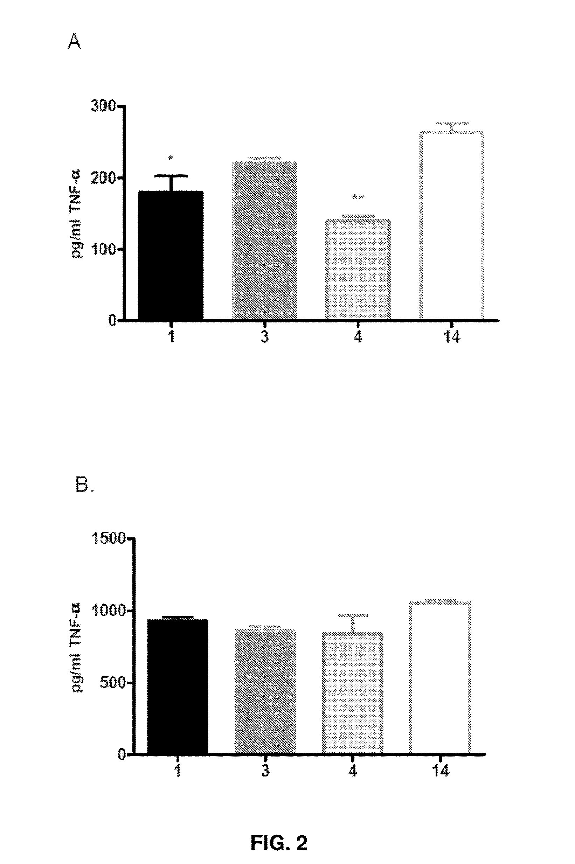

FIGURE 2 shows the *in vivo* inhibitory activity of anti-TNF-α siRNA conjugated with acridine or quindoline, using protocols A and B in HeLa cells. 1. Unmodified siRNA, 3. siRNA conjugated with acridine in the 3' end of the sense strand, 4. siRNA conjugated with quindoline in the 3' end of the sense strand, 14. siRNA with random sequence used as a negative control. The description of the sequences 1, 3, 4 and 14 of the siRNAs is detailed in Table 1 and the chemical structure of the changes is shown in schemes 1 and 2, The data represent the means ± SE, n=3 and are compared with a random sequence. **p<0.01, *p<0.05. The statistical analysis was carried out using the ANOVA method with the Bonferroni treatment.

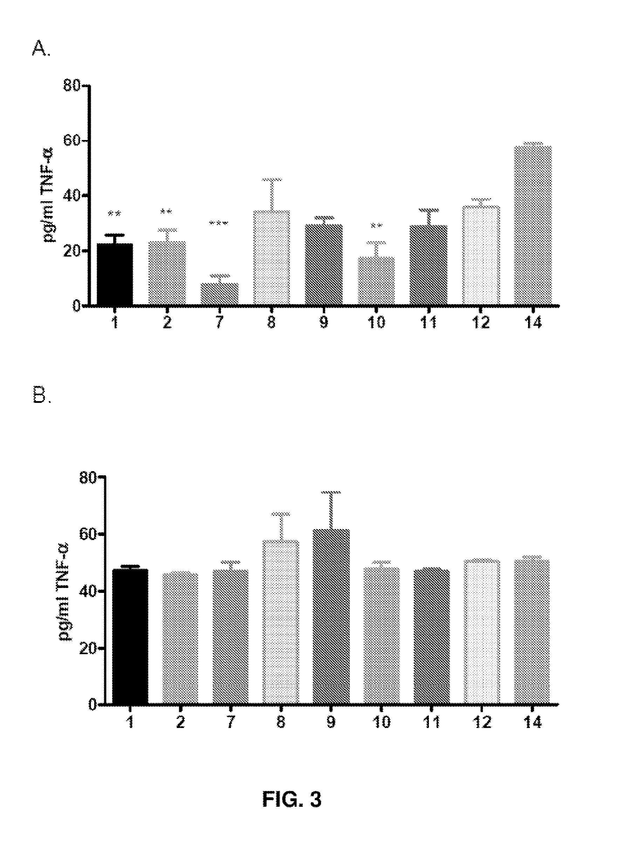

FIGURE 3 shows the effects of the formulation object of the invention on the *in vivo* inhibitory activity of several anti TNF-α siRNAs conjugated with C₁₄ and C₁₈ lipids in 4T1 cells. 1. Unmodified siRNA, 2. siRNA conjugated with cholesterol in the 3' end of the complementary strand, 7. siRNA conjugated with a C₁₄ lipid in the 3' end of the complementary strand, 8. siRNA conjugated with a C₁₈ lipid in the 3' end of the complementary strand, 9. siRNA conjugated with C₁₄ lipid in the 3' end of the guide strand, 10. siRNA conjugated with C₁₈ lipid in the 3' end of the guide strand, 11. siRNA conjugated with a C₁₄ lipid in the 3' end of the complementary strand and 2'-OMe units, 12. siRNA conjugated with C₁₄N in the 5' end of the complementary strand, 14. siRNA with random sequence used as control sequence. The description of the sequences 1, 2, 7, 8, 9, 10, 11, 12 and 14 of the siRNAs is detailed in Table 1 and the chemical structure of the changes is shown in schemes 1 and 2, The data represent the means ± SE, n=3 and are compared with a random sequence. **p<0.001, **p<0.01. The statistical analysis was carried out using the ANOVA method with the Bonferroni treatment.

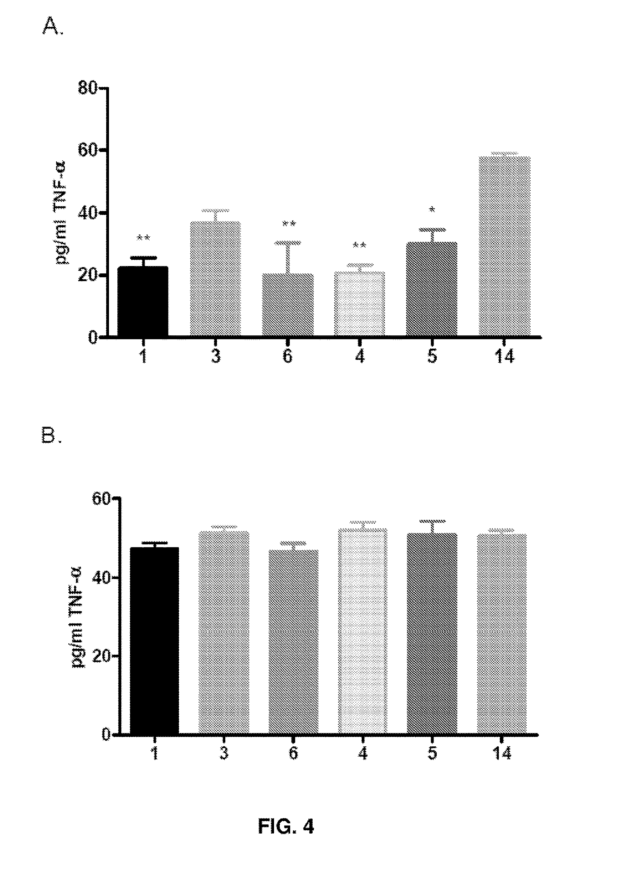

FIGURE 4 shows the effect of the formulation object of the invention on the *in vivo* inhibitory activity of anti TNF-α siRNA conjugated with acridine or quindoline, using the protocols A and B in HeLa cells. 1. Unmodified siRNA, 3. siRNA conjugated with acridine in the 3' end of the complementary strand, 6. siRNA conjugated with acridine in the 3' end of the guide strand, 4. siRNA conjugated with quindoline in the 3' end of the complementary strand, 5. siRNA conjugated with quindoline in the 3' end of the guide strand, 14. siRNA with random sequence used as a negative control. The description of the sequences 1, 3, 6, 4, 5 and 14 of the siRNAs is detailed in Table 1 and the chemical structure of the changes is shown in schemes 1 and 2, The data represent the means ± SE, n=3 and are compared with the random sequence. **p<0.01, *p<0.05. The statistical analysis was carried out using the ANOVA method with the Bonferroni treatment.

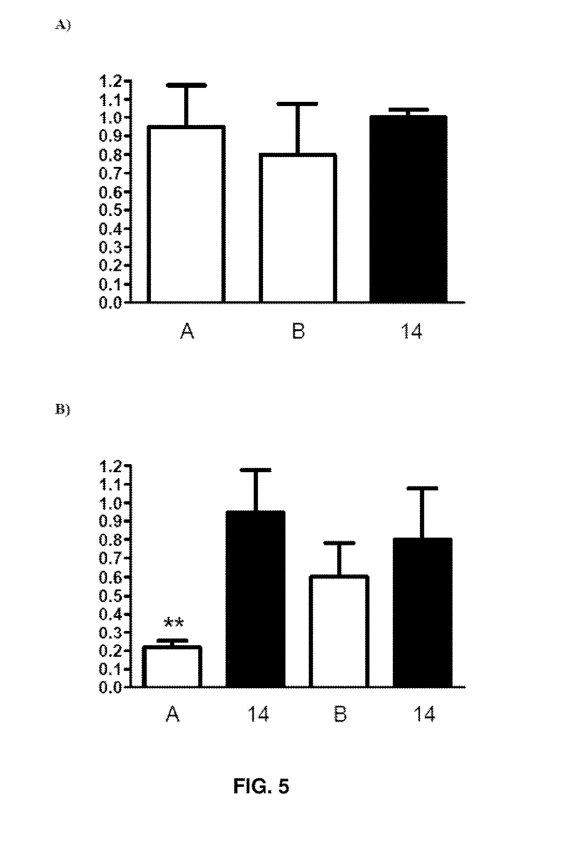

FIGURE 5 shows the effects of the formulation object of the invention on the *in vivo* inhibitory activity of siRNA against PEPCK in FAO cells. A. Unmodified siRNA, B. siRNA conjugated with cholesterol in the 3' end of the complementary strand, 14. siRNA with random sequence used as a negative control. The description of the sequences A, B and 14 of the siRNAs is detailed in Table 1 and the chemical structure of the changes is shown in schemes 1 and 2. The amounts of mRNA produced by the cells after 48 hours of treatment with the siRNAs were determined. The data represent the means ± SE, n=3 and are compared with a random sequence. **p<0.01, *p<0.05. The statistical analysis was carried out using the ANOVA method with the Bonferroni treatment.

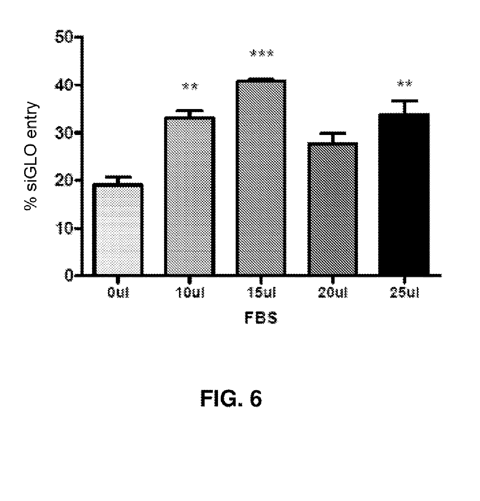

FIGURE 6 shows the measurement of the fluorescence of the siRNAs siGLO in 4T1 cells by Flow cytometry. 4T1 cells were transfected with 100 nM of siRNA siGLO (Cy3) incubated with different amounts of foetal bovine serum (0 µL to 25 µL) using ultrasound with the Protocol that we have called the formulation object of the invention. After 24 hours of incubation, the cells were analyzed in a flow cytometer. The cell entry of the Cy3-labeled siRNA was quantified by the measurement of the signal of relative fluorescence in the Cy3 channel. The cells without siRNA were used as control of fluorescence. The data represent the means ± SE, n=3 and are compared with cells transfected with the siRNAs without FBS. ***p<0.001, **p<0.01. ANOVA Test, Bonferroni post-test.

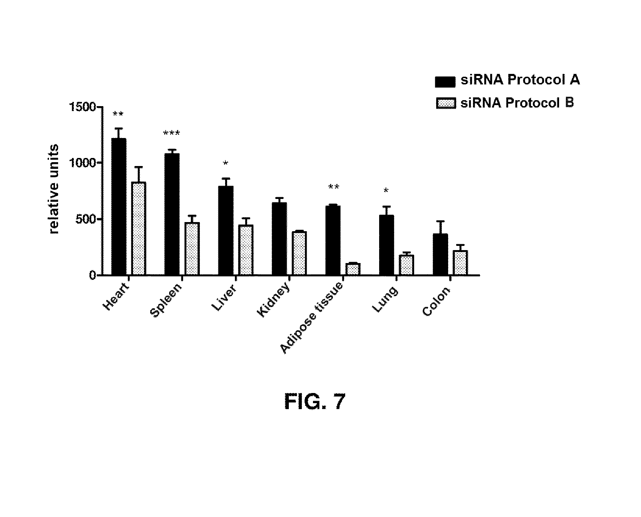

FIGURE 7 shows the biodistribution of a fluorescent siRNA. Five 8-week old mice of the ICR strain were used. 2 mice were injected via i.v. with 10 µg of siRNA siGLO according to the formulation object of the invention with 450 µL of mouse serum, other 2 mice were given the siRNA siGLO dissolved directly in 450 µL of physiological saline via i.v. and 1 mouse that was not injected was used as a control of fluorescence. After 4 hours, the animals were sacrificed and the organs were perfused in 4% PFA for subsequently cut the tissues and view them under the confocal microscope. The data represent the means ± SE, n=3 *stacks* of photos of each tissue and the formulation object of the invention is compared with the siRNA siGLO dissolved directly in the serum, between each of the tissues. ***p<0.001, **p<0.01, *p<0.05 ANOVA Test, Bonferroni post-test.

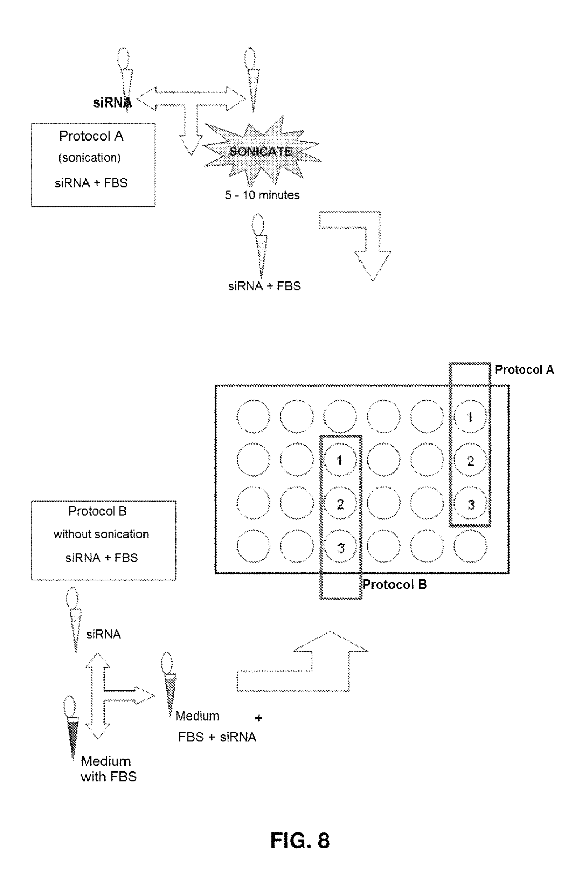

FIGURE 8 shows a diagram of the process for the formulation of the dispersion of the siRNA and the components of the serum through the use of sonication. Throughout the description and the claims the word "comprises" and its variants are not intended to exclude other technical features, additives, components or steps. For the persons skilled in the art, other objects, advantages and features of the invention will be evident in part from the description and in part from the practice of the invention. The following examples and figures are provided by way of illustration, and are not intended to be limiting of the present invention.

### EXAMPLES

### Synthesis of oligonucleotides

The oligoribonucleotides were prepared using a commercial synthesiser from Applied Biosystems, model 3400, using 2-cyanoethyl phosphoramidites and tert-butyldimethylsilyl (TBDMS) protectors for protecting the hydroxyl group in position 2'. The following solutions were used: 0.4 M 1H-tetrazole in ACN (catalyst); 3% trichloroacetic acid in DCM (detritylation); acetic anhydride /pyridine / tetrahydrofuran (1: 1: 8) (capping A); 10% N-methylimidazole in tetrahydrofuran (capping B); 0.01 M iodine in tetrahydrofuran / pyridine / water (7: 2: 1) (oxidation). The last DMT was removed in the RNA sequences because the DMT group is not fully stable to fluoride treatment. The average yield per nucleotide addition stage was 97 - 98% for RNA monomers. The polymeric supports obtained were treated with a concentrated solution of ammonia-ethanol (3: 1) for 1 hour at 55 °C. The supports were washed with ethanol and the resulting solutions combined and evaporated to dryness. The resulting products were treated with 0.15 ml of triethylamine trishydrofluoride /triethylamine / N-methylpyrrolidone (4: 3: 6) for 2.5 h at 65 °C in order to remove the TBDMS groups. The reactions were stopped by the addition of 0.3 ml isopropoxytrimethylsilane and 0.75 ml ether. The resulting mixtures were stirred and cooled to 4 °C. A precipitate formed that was centrifuged at 7000 rpm for 5 min at 4 °C. The precipitates were washed with ether and centrifuged again. The residues were dissolved in water and the conjugates were purified by HPLC. Column: Nucleosil 120 - 10 C18 (250 x 4 mm); a linear gradient of 20 min from 0% to 50% B was used with a flow of 3 ml/min. The composition of the solutions used in the HPLC was: solution A: 5% acetonitrile (ACN) in 100 mM triethylammonium acetate (pH 6.5) and solution B: 70% ACN in 100 mM triethylammonium acetate (pH 6.5). The purified products were analysed by MALDI-TOF mass spectrometry.

The MALDI-TOF spectra were obtained using a Perspective Voyager DETMRP mass spectrometer equipped with a 337 nm nitrogen laser using a 3 ns pulse. The matrix used contained 2,4,6-trihydroxyacetophenone (THAP, 10 mg/ml in ACN / water, 1: 1) and ammonium citrate (50 mg/ml in water).

### Oligoribonucleotides

The following RNA sequences were obtained from commercial sources (Sigma-Proligo, Dharmacon): negative control complementary strand 5'-CAG UCG CGU UUG CGA CUG G-dT-dT-3' (SEQ ID NO: 3); negative control guide strand 5'-CCA GUC GCA AAC GCG ACU G-dT-dT-3' (SEQ ID NO: 4); anti-TNFα guide strand (SEQ ID NO: 1); and anti-TNFα complementary strand (SEQ ID NO: 2). The RNA-type monomers are listed in uppercase, the abbreviation dT stands for thymidine. The anti-sense TNF-α strand with cholesterol at the 3' end: SEQ ID NO: 1-cholesterol was prepared using the commercial support cholesterol-tetraethylene glycol (TEG)-3'-CPG (Glen Research). The fluorescent siRNA labelled with Cy3 was obtained from commercial sources (siGLO, Thermo Scientific, Dharmacon, USA). The anti-TNF-α siRNA sequences have been described in the literature to inhibit the mRNA of TNF-α of mice [D.R. Sorensen et al. Gene silencing by systemic delivery of synthetic siRNA in adult mice. Journal of Molecular Biology 327 (2003) pages 761 - 766].

### Cell cultures, transfection and cell assays

HeLa cells were cultured in normal conditions: 37 °C, 5% CO2, modified Dulbecco Eagle medium, 10% foetal bovine serum, 2 mM L-glutamine, supplemented with penicillin (100 U/ml) and streptomycin (100 mg/mL). All the experiments were carried out at a confluence of 40 - 60%. The HeLa cells were transfected with 250 ng of plasmid expressing the mouse TNF-α gene using lipofectin (Invitrogen) and following the manufacturer's instructions.
Mouse 4T1 cells were cultured in normal conditions. 100 nM of each of the siRNA duplexes were incubated with 10% foetal bovine serum (FBS) before addition to the 4T1 cells. After 24 h, the amount of TNF-α produced by the cells was measured by ELISA assay.

### Practical example of the method of the invention

i) The siRNAs with the general formula (N19TT/N19TT) were added to foetal bovine serum in a proportion of 0.33 µg siRNA/25 µl of serum.
ii) The siRNA/serum complex was placed in an ultrasonic bath for 5 - 10 minutes.
(iii) The ultrasonically treated siRNA/serum complex was added to the cell culture in the presence of fresh cell culture medium.

### 1) Synthesis of oligoribonucleotides containing hydrophobic molecules for use in the RNA interference assay.

The following genes coding for the tumour necrosis factor (TNF-α) were selected as the target genes: 1) anti-TNFα guide strand SEQ ID NO: 1 and 2) anti-TNFα complementary strand SEQ ID NO: 2. This siRNA duplex (anti-TNF-α) has been previously described to efficiently regulate the mouse TNF-α mRNA [D.R. Sorensen et al. Gene silencing by systemic delivery of synthetic siRNA in adult mice. Journal of Molecular Biology 327 (2003) pages 761 - 766]. 12 duplexes were prepared with various hydrophobic compounds at the ends, either in the guide strand or in the complementary strand (Table 1).

The following sequences were selected as genes coding for PEPCK-C. 1) anti-PEPCK-C anti-sense or guide strand: SEQ ID NO: 5 and 2) anti-PEPCK-C sense or complementary strand: SEQ ID NO: 6. These sequences have not been described before.

Seven conjugated oligonucleotide sequences were prepared with C₁₄ and C₁₈ derivatives: two guide strands and five complementary strands conjugated with C₁₄ and C₁₈ on the 3' end (see scheme 1 and Table 1).

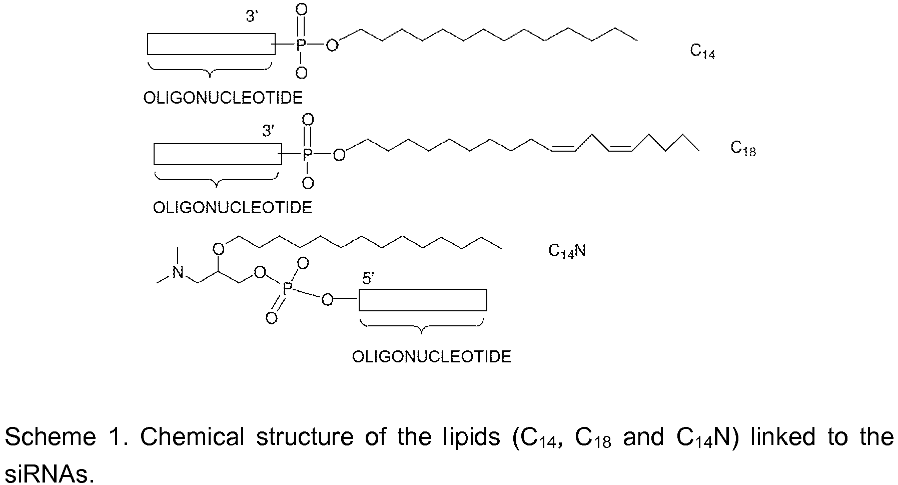

The oligonucleotides corresponding to the complementary strand 7 and 8 have C₁₄ and C₁₈ derivatives on the 3' end respectively. Oligonucleotide 12 has a C₁₄N derivative on the 5' end (Table 1). Oligonucleotide 11 has a C₁₄ derivative on the 3' end and various 2'-O-methyl units along the complementary strand except on the last two thymidines. It has been reported that 2'-O-methyl units stabilise the RNA strands from nuclease degradation. The position of the 2'-O-methyl units (mC = 2'-O-methyl-C and mU = 2'-O-methyl-U) in oligonucleotide 11 is 5'-GUG C (mC) U AUG (mU) (mC) U (mC) AG (mC) C (mu) CTT-3' (SEQ ID NO: 7)-C₁₄.

Finally four conjugated oligonucleotides were prepared with acridine or quindoline at the 3' end (Table 1). Two complementary strands conjugated with acridine 3 or quindoline 4 respectively and two guide strands conjugated with acridine 6 or quindoline 5. The guide strand conjugated with cholesterol in the 3' end was prepared using commercial reagents. The synthesis of the RNA sequences was carried out by the patent inventors.

**Table 1. Sequences of the siRNAs containing hydrophobic molecules.**

| Code | Modification | Sequence (TNFα) | |
|---|---|---|---|
| 1 | unmodified anti-TNFα | G | GAGGCUGAGACAUAGGCACTT (SEQ ID NO: 1) |
| | | A | GUGCCUAUGUCUCAGCCUCTT (SEQ ID NO: 2) |
| 2 | Complementary 3'-cholesterol | G | SEQ ID NO: 1 |
| | | A | SEQ ID NO: 2 - cholesterol |
| 3 | complementary 3'-acridine | G | SEQ ID NO: 1 |
| | | A | SEQ ID NO: 2 - acridine |
| 4 | complementary 3'-quindoline | G | SEQ ID NO: 1 |
| | | A | SEQ ID NO: 2 - quindoline |
| 5 | guide 3'-quindoline | G | SEQ ID NO: 1 - quindoline |
| | | A | SEQ ID NO: 2 |
| 6 | guide 3'-acridine | G | SEQ ID NO: 1 - acridine |
| | | A | SEQ ID NO: 2 |
| 7 | complementary C14 lipid 3' | G | SEQ ID NO: 1 |
| | | A | SEQ ID NO: 2 - C14 |
| 8 | complementary C18 lipid, 3' | G | SEQ ID NO: 1 |
| | | A | SEQ ID NO: 2 - C18 |
| 9 | guide C14 lipid, 3' | G | SEQ ID NO: 1 - C14 |
| | | A | SEQ ID NO: 2 |
| 10 | guide C18 lipid, 3' | G | SEQ ID NO: 1 - C18/ |
| | | A | SEQ ID NO: 2 |
| 11 | complementary C14 lipid + OMe, 3' | G | SEQ ID NO: 1 |
| | | A | GUGC(mC)UAUG(mU)(mC)U(mC)AG(mC)C(mU)CT T (SEQ ID NO: 7) - C14 |
| 12 | complementary C14N lipid, 5' | G | SEQ ID NO: 1 |
| | | A | C14N - SEQ ID NO: 2 |
| 14 | unmodified random control | G | CAGUCGCGUUUGCGACUGGTT (SEQ ID NO: 3) |
| | | A | CCAGUCGCAAACGCGACUGTT (SEQ ID NO: 4) |

| Code | Modification | | Sequence (PEPCK-C) |
|---|---|---|---|
| A | unmodified PEPCK-C | G | UUACAUCUGGCUGAUUCUCTT (SEQ ID NO: 5) |
| | | A | GAGAAUCAGCCAGAUGUAATT (SEQ ID NO: 6) |
| B | complementary cholesterol, 3' | G | SEQ ID NO: 5 |
| | | A | SEQ ID NO: 6 - cholesterol |

G: guide strand / A: complementary strand. The nucleotide sequences of these chains are shown from 5' to 3'. mC and mU represent the position of the 2'-O-methyl derivatives 2'-O-methyl-C and 2'-O-methyl-U respectively.

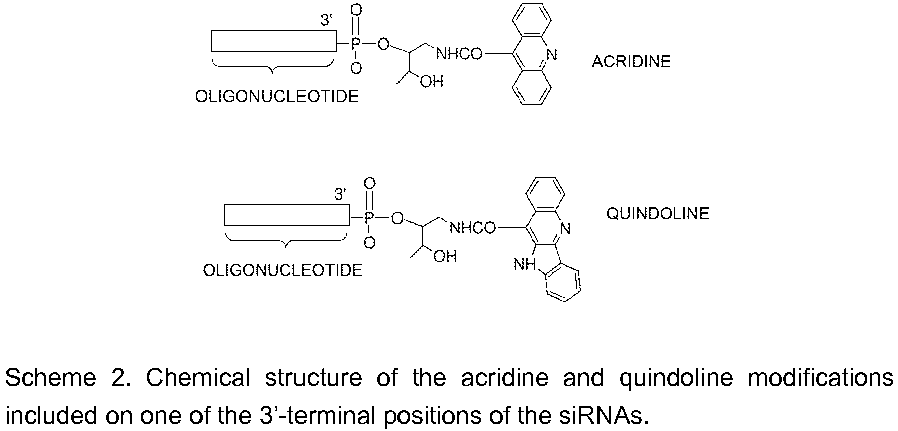

### 2) Inhibition of TNF-α. The guide and complementary strands were hybridised and the result was a duplex or siRNA that was used to inhibit the expression of the TNF-α gene. Firstly, the inhibitory properties of the siRNA were evaluated in HeLa cells with and without the formulation of the invention. These cells do not express the mouse TNF-α gene therefore a co-transfection was carried out with the pCAm plasmid containing the mouse TNF-α gene. Two different protocols were investigated.

PROTOCOL A) The HeLa cells were transfected with 250 ng of mouse TNF-α pCAm plasmid using lipofectin. After one hour the cells were treated with the siRNAs (100 nM) without using any transfection reagent, but were incubated with foetal bovine serum and treated with ultrasonification before the transfection process. After 48 h the amount of TNF-α produced by the cells was measured by ELISA.

PROTOCOL B) The HeLa cells were treated with 250 ng of the mouse TNF-α pCAm plasmid using lipofectin as described in protocol A. After one hour the cells were treated with the siRNA duplex (100 nM) in the absence of serum. After 48 h the amount of TNF-α produced by the cells was measured by ELISA.

The results of this experiment showed that the amount of TNF-α produced after 48 hours of the addition of the modified siRNA duplex incubated with 10% serum and treated with ultrasonification (A) were clearly different from the addition without incubation (B). An inhibition of between 40% and 50% in the TNF-α protein produced compared with the random sequence siRNA was observed only when the siRNA was transfected with the formulation of the invention. The presence of the lipid units on the 3' and 5' ends of the sense strand increased the inhibition of the expression of TNF-α.

Figure 2 indicates that the siRNAs conjugated with acridine or quindoline pretreated with serum and ultrasound produced an inhibition of between 30% and 50% in the production of TNF-α compared with random siRNA used as a negative control. No inhibition was observed in the absence of the formulation of the invention. Only quindoline at the 3' end of the sense strand increased the inhibition in the expression of TNF-α.

The inhibitory properties of the siRNAs in mouse 4T1 cells were studied next. These cells produce endogenous mouse TNF-α. In these experiments, the cells were treated with the siRNA duplex (100 nM) without the use of any transfection reagent. The siRNAs were incubated with serum and ultrasound or simply added to the cells without prior treatment. After 24 h the amount of TNF-α produced by the cells was measured by ELISA.

The results of the inhibition of TNF-α production by siRNAs conjugated with lipids is shown in Figure 3. As described for the HeLa cells, there was strong inhibition in the production of TNF-α only when the formulation of the invention was used. The highest inhibition (80%) was observed when using the siRNA duplex conjugated with lipid C₁₄ at the 3' end of the complementary strand (7). The results of the inhibition of TNF-α production by siRNAs conjugated with acridine or quindoline are shown in Figure 4. The guide and complementary strands were modified at the 3' end (3 and 6). The figure shows strong inhibition in the TNF-α production using the formulation of the invention. The highest inhibition (60%) was observed with siRNA conjugated with quindoline at the 3' end of the complementary strand (4).

### 3) Inhibition of PEPCK-C.

The guide and complementary strands complementary to PEPCK were hybridised and the resulting duplex was used to inhibit the expression of the PEPCK-C gene. The properties of the siRNA were investigated in FAO cells (rat hepatoma) with and without the formulation object of the invention. These cells express the PEPCK-C gene endogenously (pck1); therefore it was not necessary to carry out co-transfection with the corresponding plasmid. The following protocols were used:
A) FAO cells were treated with the siRNA duplex (100 nM) in the absence of serum. After 48 hours the amount of PEPCK-C mRNA produced by the cells was measured by qRT-PCR.
B) FAO cells were treated with the siRNA duplex (100 nM) incubated with foetal bovine serum and ultrasound. After 48 hours the amount of PEPCK-C mRNA produced by the cells was measured by qRT-PCR.

The results of this experiment demonstrate that the siRNA incubated with serum and ultrasound significantly improved the gene silencing of the target Pck1 gene in the FAO cells. It is significant that the improvement of the entry of the siRNA by action of the formulation object of the invention did not require modifications in the siRNA to help increase the efficiency. Using this method, the unmodified siRNA is a good inhibitor of the PEPCK-C mRNA.

### 4) Cellular entry of a siRNA labelled with a fluorescent molecule (Cy3) using the formulation object of the invention.

The method was optimised, assuming that the efficiency of the method depended on the interaction of the siRNA with the plasma components, by determining the best siRNA/serum ratio. To do this, mouse 4T1 cells were transfected with a fluorescent siRNA labelled with Cy3 previously incubated with different volumes (from 0 µL to 25 µL) of foetal bovine serum and treated by ultrasonification. The entry of siRNA into the cells was measured by flow cytometry (DakoCytomation, model MoFlo) at a speed of 1000 cells per second.

The images obtained using flow cytometry show that when the siGLO siRNA was directly transfected on the cells with medium supplemented with serum, that is without the formulation object of the invention, only 20% of the cells in the field were transfected, whereas in the case of the formulation object of the invention of the siGLO siRNA with 15 µL of FBS, 50% of the cells showed high fluorescence (Figure 6). According to these data, the most efficient ratio to ensure highest entry of siRNA into the cells is 0.33 µg siRNA/15 µL serum, which is the concentration used in the *in vivo* trials.

It was also observed that when increasing the volume of serum, the efficiency of entry of siRNA into the cells was reduced. This could be due to a possible saturation of the binding sites of the proteins in the serum.

### In vivo trials

### 5) In vivo delivery of siGLO siRNA in mice.

Five 8-week old mice of the ICR strain (Harlan, Interfauna Ibérica, Spain) were used. The animals were housed in standard environmental conditions, temperature 22 °C, relative humidity 70 - 80% and with a 12-hour daily light cycle until the time of the trials.

All the protocols used were previously approved by the ethics committee of the University of Barcelona and the manipulation of the animals was governed by European Community regulations.

The trial consisted of administering by injection into the tail vein of mice, 450 µL of a solution of the Cy3 fluorescent siRNA, incubated with ultrasound or not with mouse serum, keeping a ratio of 0.33 µg siRNA:15 µL serum. After 4 hours, the animals were sacrificed with ether and the tissues were perfused with 4% PFA. Next the tissues were cut in a cryostat (LEICA) into slices of size 30 µm to be mounted on slides and observed under a confocal microscope (Leica model TCS NT). Three photographs of longitudinal slices of each of the organs were taken, keeping the same acquisition parameters between the organs. The fluorescence observed in the photographs was quantified using a computer program, MacBiophotonics ImageJ that allows obtaining quantitative values of the fluorescence in the tissues.

As shown in Figure 7, 4 hours after i.v. administration the levels of fluorescence of the siGLO without the formulation of the invention were lower in all the organs whereas significant levels of between 20% and 50% higher for siRNA were found in the lung, heart, liver, spleen and adipose tissue when preincubated with mouse serum. This indicates that the formulation object of the invention improves the bio-distribution properties of the siRNAs.

## Claims

1. A process for obtaining a formulation comprising siRNAs associated with plasma components, **characterized in that** the process comprises a step in which the plasma components are dispersed in aqueous medium.

2. The process according to the previous claim, **characterized in that** the dispersion of the plasma components is carried out using ultrasound.

3. The process according to any of the previous claims, **characterized in that** siRNAs and plasma components ratio varies between 1:100 and 1:50000.

4. The process according to the previous claim, **characterized in that** the siRNAs and plasma components ratio varies between 1:500 and 1:10000.

5. The process according to the previous claim, **characterized in that** the siRNAs and plasma components ratio varies between 1:3000 and 1:5000.

6. The process according to any of the previous claims, **characterized in that** blood plasma and/or serum are used as source of plasma components.

7. The process according to any of the previous claims, **characterized in that** the formulation is injectable.

8. The process according to any of the previous claims, **characterized in that** the siRNA is not derivatized at its 3'- or 5'- end in any of its strands.

9. The process according to the previous claim, **characterized in that** the siRNA is from a natural source (isolated from cells), or is obtained by enzymatic copying process of a DNA template through the use of RNA polymerase or synthesized from a template DNA through the use of RNA polymerase.

10. The process according to any of claim 1 to 7, **characterized in that** the siRNAs is derivatized at its 3' or 5' position with at least one lipid, at least one peptide, at least one carbohydrate or any combination thereof.

11. The process according to any of the previous claims, **characterized in that** the siRNA comprises between 15 and 40 nucleotides.

12. The process according to any of the previous claims, **characterized in that** the siRNA comprises between 19 and 25 nucleotides.

13. The process according to any of the previous claims, **characterized in that** the dispersion is carried out at a temperature between 0 and 85 °C.

14. The process according to the previous claim **characterized in that** the dispersion is carried out at a temperature between 5 and 50 °C.

15. The process according to the previous claim, **characterized in that** the dispersion is carried out at a temperature between 10 and 35 °C.

16. The process according to any of the previous claims, **characterized in that** the dispersion is carried out at least for 1 second.

17. The process according to the previous claim, **characterized in that** the dispersion is carried out at least for 15 seconds.

18. The process according to the previous claim, **characterized in that** the dispersion is carried out for at least between 30 seconds and 2 hours.

19. The process according to the previous claim, **characterized in that** the dispersion is carried out for at least between 60 seconds and 30 min.

20. The process according to any of the previous claims, **characterized in that** siRNA duplex comprises at least one substitution selected from 2'-O-methyl-ribonucleotide; 2'-deoxyribonucleotide; 2'-methoxyethyl-ribonucleotide; 2'-fluoro-deoxyribonucleotide; 2'-fluoro-arabinonucleotide; conformationally restricted nucleosides; RNA with phosphorothioate bonds; abasic moieties or ribitol; or nucleosides containing modified bases such as 5-methylcytosine, 5-methyluracil, 4-alkynylcytosine, 5-alkynyluracil, 5-halogencytosine, 5-halogenuracil, 7-deazadenine, 7-deazaguanine or hypoxanthine.

21. The process according to any of the previous claims, **characterized in that** the siRNA duplex partially or completely inhibits/silences the expression of at least one of the following genes: tumor necrosis factor alpha (TNFα), vascular endothelial growth factor (VEGF), VEGF receptor (VEGF R1/2), granulocyte (GM-CSF-1) and macrophages (M-CSF-1) colony suppressor factor, angiopoietin (ANGPT), apolipoprotein (ApoB), choroidal neovascularization (CNV), phosphoenolpyruvate carboxykinase (PEPCK), human epidermal growth factor receptor (HER2), macrophage inflammatory protein (MIP2), N-methyl-D aspartate receptor (NMDA), keratinocyte-derived cytokines (KC), delta opioid receptor (DOR), Discoidin domain receptor (DDR1), PIV phosphoprotein gene (PIV-P), heme oxygenase (HMOX1), caveloin, dopamine transporter, Green fluorescent protein and Swing sarcoma protein (EWS-FL/1).

22. The process according to the previous claim, **characterized in that** the siRNA duplex (Y) partially or completely inhibits/silences the expression of at least one of the following genes: tumor necrosis factor alpha (TNFα), vascular endothelial growth factor (VEGF), VEGF receptor (VEGF R1/2), granulocyte (GM-CSF-1) and macrophages (M-CSF-1) colony suppressor factor.

23. The process according to any of the previous claims, **characterized in that** the siRNA sequence is selected from: antisense or guide anti- TNFα 5'SEQ ID NO: 1; sense or complementary anti- TNFα: SEQ ID NO: 2; antisense or guide strand anti-PEPCK-C: SEQ ID NO: 5; SEQ ID NO: 6.

24. A pharmaceutical composition of siRNA associated with plasma components obtainable by any of the processes defined in the previous claims.

25. The pharmaceutical composition according to the previous claim, **characterized in that** it comprises at least one pharmaceutically acceptable excipient or vehicle.

26. The pharmaceutical composition according to the previous claim, **characterized in that** it comprises at least one transfection agent.

27. The pharmaceutical composition according to the previous claim, **characterized in that** the transfection agent is selected from the list comprising the following compounds: lipofectin, lipofectamine, oligofectamine, effectene, cellfectin, DOTAP, DOPE, fugene, polyethylene glycol, cholesterol, polyethylenimide (PEI), Jet-polyethylenimide, cell penetrating peptides, trojan peptides, TAT peptide, penetratin, oligoarginine, poly-lysine, rabies virus glycoprotein, gold nanoparticles, dendrimers, carbon nanotubes, cationic lipids and liposomes.

28. The pharmaceutical composition according to any of claims 24 to 27, where it occurs in a form adapted to the oral or parenteral administration, preferably parenteral.

29. The use of the pharmaceutical compositions according to any of the five previous claims for the preparation of a medicament.

30. The use according to the previous claim for the preparation of a medicament for gene silencing.

31. The use according to any of the two previous claims for the preparation of a drug for the treatment of cancer, inflammation, colitis, ulcerative colitis, Crohn's disease and rheumatoid arthritis.
